# EUROPEAN PATENT APPLICATION

(11) **EP 3 889 613 A1**
(43) Date of publication of application: **06.10.2021**
(21) Application number: 21163690.7
(22) Date of filing: 19.03.2021
(51) Int. Cl.: G01N 33/86, G01N 33/49, C07K 14/755

(54) **METHOD FOR MEASURING A BLOOD COAGULATION ACTIVITY**

(30) Priority: 30.03.2020 JP 2020059935
(71) Applicant: SYSMEX CORPORATION, Kobe-shi Hyogo 651-0073 (JP); Keio University, Tokyo, 108-8345 (JP); Social Welfare Organization Saiseikai Imperial Gift Foundation, Inc., Tokyo 108-0073 (JP)
(72) Inventor: Shinohara, Sho, Hyogo, 651-0073 (JP); Kumano, Osamu, Hyogo, 651-0073 (JP); Arai, Nobuo, Hyogo, 651-0073 (JP); Madoiwa, Seiji, Tokyo, 108-0073 (JP); Fujimori, Yuta, Tokyo, 108-8345 (JP)
(74) Representative: Hoffmann Eitle

(57) **Abstract**

Disclosed is a method for measuring a blood coagulation activity, comprising:
acquiring a parameter related to differentiation of a coagulation waveform in a blood specimen collected from a patient administered with
(1) a polypeptide containing a sequence represented by SEQ ID NO: 1, or
(2) a polypeptide containing a polypeptide having 95% or more identity with the sequence represented by SEQ ID NO: 1 and having an activity as a blood coagulation factor VIII; and

acquiring an activity value of a blood coagulation factor VIII in the blood specimen based on the acquired parameter.

## Description

### FIELD OF THE INVENTION

The present specification discloses a method for measuring a blood coagulation activity.

### BACKGROUND

Lonoctocog alfa described in U.S. Patent No. 7041635 is a factor VIII analog, one of genetically modified human blood coagulation factors, and is used for suppressing bleeding tendency in patients with factor VIII deficiency.

Coagulation activity in a patient administered with Lonoctocog alfa is generally measured and monitored by one-step coagulation method such as activated partial thromboplastin time (APTT). However, it is described that when activity of Lonoctocog alfa is measured after administration by the one-step coagulation method, a measurement result apparently shows a low value in AFSTYLA (trademark) HIGHLIGHTS OF PRESCRIBING INFORMATION (AFSTYLA (trademark) HIGHLIGHTS OF PRESCRIBING INFORMATION (https://labeling.cslbehring.com/PI/US/Afstyla/EN/Afstyla-Prescribing-Information.pdf) (hereinafter, referred to as Publication 1)). Therefore, in Publication 1, when the activity of Lonoctocog alfa in plasma was measured by the one-step coagulation method, it is instructed to multiply a measured value of factor VIII obtained by the one-step coagulation method by conversion factor 2 to be a measured value of factor VIII of the patient.

However, in order to follow instructions described in Publication 1, it is necessary to identify a specimen of a patient to whom Lonoctocog alfa has been administered from many measured values and multiply the identified specimen by a conversion factor, which is complicated to process and also may cause human error.

An object of the present invention is to provide a method for measuring blood coagulation activity, which can facilitate measurement of a specimen of a patient to whom Lonoctocog alfa has been administered and also can suppress human error.

### SUMMARY OF THE INVENTION

One embodiment of the present invention relates to a method for measuring a blood coagulation activity. The measurement method includes acquiring a parameter related to differentiation of a coagulation waveform in a blood specimen collected from a patient administered with (1) a polypeptide containing a sequence represented by SEQ ID NO: 1, or (2) a polypeptide containing a polypeptide having 95% or more identity with the sequence represented by SEQ ID NO: 1 and having an activity as a blood coagulation factor VIII, and acquiring an activity value of the blood coagulation factor VIII in the blood specimen based on the acquired parameter.

According to this embodiment, the blood coagulation factor VIII activity of Lonoctocog alfa can be measured more accurately.

It is possible to provide a method for measuring blood coagulation activity, which can facilitate measurement of a specimen of a patient to whom Lonoctocog alfa has been administered and also can suppress human error.

### BRIEF DESCRIPTION OF THE DRAWINGS

Fig. 1A shows an example of a coagulation waveform. Fig. 1B shows an example of a corrected coagulation waveform.
Figs. 2A to 2C show factor VIII activities of each preparation calculated based on coagulation time. Fig. 2A shows activity values when each preparation was added to factor VIII deficient plasma so as to be 1.00 IU/dL. Fig. 2B shows activity values when each preparation was added to factor VIII deficient plasma so as to be 0.30 IU/dL. Fig. 2C shows activity values when each preparation was added to factor VIII deficient plasma so as to be 0.05 IU/dL.
Fig. 3A shows parameter values of ADVATE and AFSTYLA. Fig. 3B shows first differential waveforms of APTT coagulation waveforms of ADVATE and AFSTYLA. Fig. 3C shows second differential waveforms of APTT coagulation waveforms of ADVATE and AFSTYLA.
Fig. 4A shows first differential waveforms of corrected APTT coagulation waveforms of ADVATE and AFSTYLA. Fig. 4B shows second differential waveforms of corrected APTT coagulation waveforms of ADVATE and AFSTYLA.
Fig. 5A shows a calibration curve of min1. Fig. 5B shows a calibration curve of min2.
Fig. 6A shows a calibration curve of Max2. Fig. 6B shows a calibration curve of Ad|min1|.
Fig. 7A shows activity values (IU/dL) of AFSTYLA obtained based on parameter values related to differentiation of each APTT coagulation waveform. Fig. 7B shows addition recovery rates (%).
Figs. 8A to 8C show activity values of factor VIII of each preparation calculated based on maximum coagulation rate min1. Fig. 8A shows activity values when each preparation was added to factor VIII deficient plasma so as to be 1.00 IU/dL. Fig. 8B shows activity values when each preparation was added to factor VIII deficient plasma so as to be 0.30 IU/dL. Fig. 8C shows activity values when each preparation was added to factor VIII deficient plasma so as to be 0.05 IU/dL.
Figs. 9A to 9C show activity values of factor VIII of each preparation calculated based on corrected maximum coagulation rate Ad|min1|. Fig. 9A shows activity values when each preparation was added to factor VIII deficient plasma so as to be 1.00 IU/dL. Fig. 9B shows activity values when each preparation was added to factor VIII deficient plasma so as to be 0.30 IU/dL. Fig. 9C shows activity values when each preparation was added to factor VIII deficient plasma so as to be 0.05 IU/dL.
Figs. 10A to 10C show activity values of factor VIII of each preparation calculated based on maximum coagulation acceleration min2. Fig. 10A shows activity values when each preparation was added to factor VIII deficient plasma so as to be 1.00 IU/dL. Fig. 10B shows activity values when each preparation was added to factor VIII deficient plasma so as to be 0.30 IU/dL. Fig. 10C shows activity values when each preparation was added to factor VIII deficient plasma so as to be 0.05 IU/dL.
Figs. 11A to 11C show activity values of factor VIII of each preparation calculated based on maximum coagulation deceleration max2. Fig. 11A shows activity values when each preparation was added to factor VIII deficient plasma so as to be 1.00 IU/dL. Fig. 11B shows activity values when each preparation was added to factor VIII deficient plasma so as to be 0.30 IU/dL. Fig. 11C shows activity values when each preparation was added to factor VIII deficient plasma so as to be 0.05 IU/dL.

### DETAILED DESCRIPTION OF THE PREFERRED EMBODIMENTS

### 1. Explanation of terms

In the present specification, a subject for acquiring an activity value of blood coagulation factor VIII is a polypeptide having an activity as blood coagulation factor VIII containing a predetermined amino acid sequence. In the present specification, blood coagulation factor VIII may be simply referred to as "factor VIII", and a polypeptide having activity as blood coagulation factor VIII may be simply referred to as "polypeptide".

Preferably, the polypeptide is a polypeptide containing a sequence represented by SEQ ID NO: 1. The polypeptide containing a sequence represented by SEQ ID NO: 1 may be modified by disulfide bonding, sugar chain bonding, sulfation, or the like.

For example, a disulfide bonding can be formed between at least one selected from between a cysteine residue at position 153 and a cysteine residue at position 179, between a cysteine residue at position 248 and a cysteine residue at position 321, between a cysteine residue at position 528 and a cysteine residue at position 554, between a cysteine residue at position 630 and a cysteine residue at position 711, between a cysteine residue at position 944 and a cysteine residue at position 971, between a cysteine residue at position 1111 and a cysteine residue at position 1115, between a cysteine residue at position 1131 and a cysteine residue at position 1281, and between a cysteine residue at position 1286 and a cysteine residue at position 1438 of SEQ ID NO: 1.

At least one asparagine residue selected from positions 71, 239, 757, 764, 922, and 1230 of SEQ ID NO: 1 can be bonded with a sugar chain.

At least one selected from a serine residue at position 741, a serine residue at position 743, a serine residue at position 746, a threonine residue at position 759, a threonine residue at position 760, a threonine residue at position 765, a threonine residue at position 766, a serine residue at position 769, and a serine residue at position 781 of SEQ ID NO: 1 can also be bonded with a sugar chain.

At least one tyrosine residue selected from positions 346, 718, 719, 723, 776, and 792 of SEQ ID NO: 1 can undergo sulfation.

As used herein, the "residue" of various amino acids is a constituent unit of amino acids constituting a polypeptide, and intends a group in which hydrogen atoms are excluded from the amino group in the main chain and/or -OH is excluded from the carboxyl group in the main chain, from the amino acids.

The polypeptide may include a polypeptide having a certain percentage or more identity with the sequence represented by SEQ ID NO: 1. The certain percentage is not limited as long as it has the same or higher activity as a factor VIII as the polypeptide containing the sequence represented by SEQ ID NO: 1. For example, 70%, 75%, 80%, 85%, 90%, 95%, 98% or 99% of the sequence represented by SEQ ID NO: 1 is intended as the certain percentage. Preferably, 95%, 98%, or 99% is intended as the certain percentage.

Examples of an amino acid substitution that maintains the same or higher activity as a factor VIII as the sequence represented by SEQ ID NO: 1 include substitution between amino acids of a class to which each amino acid belongs. For example, when the class is a non-polar (hydrophobic) amino acid, alanine, leucine, isoleucine, valine, proline, phenylalanine, tryptophan and methionine may be included. When the class is a polar neutral amino acid, glycine, serine, threonine, cysteine, tyrosine, asparagine and glutamine may be included. When the class is a basic amino acid, arginine, lysine and histidine may be included. When the class is an acidic amino acid, aspartic acid and glutamic acid may be included.

The method for measuring activity of factor VIII will be described later.

The above polypeptide can be produced by a gene recombination technique.

Amino acids constituting the polypeptide may be artificial amino acids. The polypeptide may be modified other than the above. Examples of the modification include polyethylene glycolation modification, phosphorylation modification, acetylation modification, methylation modification, fluorescent modification, biotinylation modification, sugar modification, lipid modification, acylation modification, reductive amination modification, azide modification, ketene modification, and the like.

Examples of the polypeptide most preferably include Lonoctocog alfa [trade name: AFSTYLA (trademark)] described in Publication 1.

Patients are not limited as long as they require administration of factor VIII. Examples include patients with factor VIII deficiency. Examples of the factor VIII deficiency include hemophilia A, disseminated intravascular coagulation syndrome, liver dysfunction, and the like.

A blood specimen is a blood sample collected from a patient and is not limited as long as it is a sample of which blood coagulation activity described later can be measured. As a blood sample, for example, whole blood or plasma can be exemplified. The blood sample is preferably collected using an anticoagulant other than a heparin preparation at the time of blood collection. It is more preferable to collect using a citrate, for example, a sodium citrate solution as the anticoagulant. The blood sample is most preferably plasma isolated from a whole blood sample mixed using a 3.1 to 3.3% (weight/volume) trisodium citrate solution is used as the anticoagulant so that the anticoagulant and the patient's whole blood have a volume of about 1 : 8.5 to 9.5.

### 2. How to measure blood coagulation activity

The embodiment of the present disclosure relates to a method for measuring blood coagulation activity. The measurement method can include acquiring a parameter related to differentiation of coagulation waveform in a blood specimen collected from a patient administered with (1) a polypeptide containing a sequence represented by SEQ ID NO: 1, or

(2) a polypeptide containing a polypeptide having 95% or more identity with the sequence represented by SEQ ID NO: 1 and having activity as a blood coagulation factor VIII, and acquiring an activity value of blood coagulation factor VIII in the blood specimen based on the acquired parameter, described in 1. above.

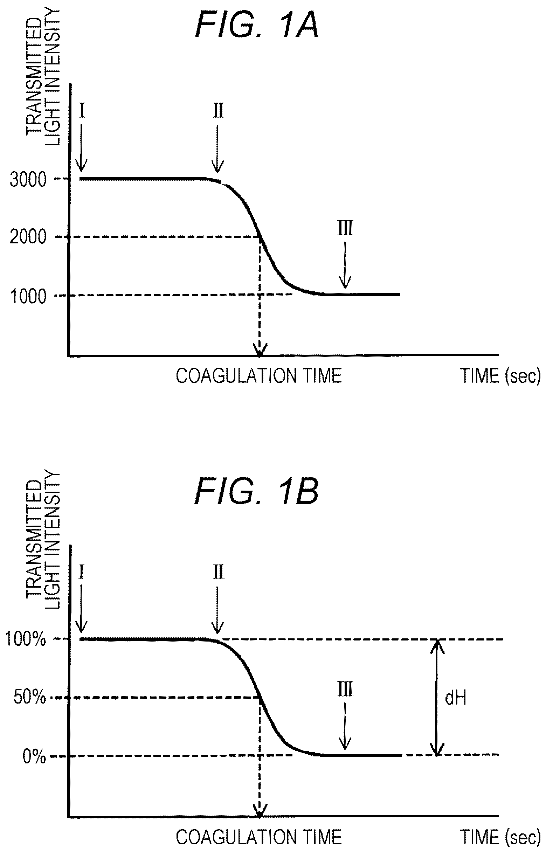

The coagulation waveform will be described with reference to Fig. 1A. The coagulation waveform shown in Fig. 1A is a coagulation waveform obtained by a method for measuring blood coagulation activity, which is generally referred to as one-step coagulation method. The one-step coagulation method is a method of measuring coagulation time by adding calcium ions required for blood coagulation and a predetermined test reagent to a blood sample containing fibrinogen whose coagulation time is to be measured (hereinafter referred to as "test sample"), irradiating a reaction solution with light, and monitoring optical change of the reaction solution with time.

Fig. 1A shows an example of a coagulation waveform. In Fig. 1A, a vertical axis (Y axis) shows transmitted light intensity, and a horizontal axis (X axis) shows measurement time (second: sec) for monitoring the transmitted light intensity. The coagulation waveform can be generated by plotting the monitored change in transmitted light intensity with time on the two axes of transmitted light intensity and measurement time. Point I in Fig. 1A is a time when the calcium ions and the test reagent were added to the test sample, and is also a time of starting the measurement (t_{I}). At the start of the measurement, the fibrinogen in the reaction solution has not changed to fibrin, and the reaction solution does not yet undergo precipitation of fibrin, so that the transmitted light intensity shows a high value. Thereafter, when the coagulation reaction proceeds and fibrin begins to precipitate, the transmitted light intensity begins to decrease. This is because the precipitated fibrin blocks light. This point is point II in Fig. 1A, which is a coagulation start time. The measurement time at which coagulation started is represented by (t_{II}). The transmitted light intensity decreases as the reaction progresses and the precipitation of fibrin progresses. When most of the fibrinogen in the test sample changes to fibrin, the reaction settles and the change in transmitted light intensity becomes a plateau. This point is point III in Fig. 1A, which is a coagulation end time. The measurement time at which coagulation finished is represented by (t_{III}). The coagulation time (CT) can generally be expressed by measurement time of CT (sec) = [(t_{III}) - (t_{II})]/2. "-" is intended for subtraction, and "/" is intended for division. That is, parameters related to coagulation activities such as coagulation start time, coagulation end time and coagulation time can be acquired from the coagulation waveform.

However, initial fibrinogen contained in the test sample may be low depending on the patient, so that it is preferable to normalize the coagulation waveform and determine the coagulation time as shown in Fig. 1B. Normalization can be achieved, for example, by assuming as 100% the amount of change in transmitted light intensity (dH), which is a difference between the transmitted light intensity at point II at the start of the coagulation reaction and the transmitted light intensity at point III at the end of coagulation, and making the change in transmitted light intensity into a relative value. In this case, the coagulation time (CT) can be set as a point at which the amount of change in transmitted light intensity (dH) is, for example, 30%, 40%, 50%, or 60%. In a preferred embodiment, the coagulation time is time at which the amount of change in transmitted light intensity (dH) is 50%.

In the present specification, the "coagulation waveform" may include a coagulation waveform generated without normalization on the monitored transmitted light intensity and a coagulation waveform generated based on corrected monitoring data obtained by performing the normalization on the monitored transmitted light intensity (hereinafter referred to as "corrected coagulation waveform"). It is preferable to use the corrected coagulation waveform as the coagulation waveform. In the present specification, the coagulation time acquired from the corrected coagulation waveform is referred to as "corrected coagulation time".

Examples of comprehensive methods for measuring a blood coagulation activity that can be measured by the one-step coagulation method include activated partial thromboplastin time (APTT), prothrombin time (PT), and the like. Various coagulation factor activities that can be measured using a comprehensive measurement method can also be measured by the one-step coagulation method. Coagulation factors whose activity can be measured using APTT are factor VIII, factor V, factor X, factor IX, factor XI, factor XII, prekallikrein, high molecular weight kininogen, prothrombin, fibrinogen, and the like. Coagulation factors whose activity can be measured using PT are factor VII, factor V, factor X, prothrombin, fibrinogen, and the like.

In this embodiment, an activity value of factor VIII is acquired. The activity value of factor VIII can be acquired using APTT. Here, "acquired" may include that the activity value is calculated or the activity value is received from another.

When measuring APTT, a predetermined test reagent is an APTT reagent that may include an activator such as silica, eladic acid or celite; an animal-derived, plant-derived, or artificially synthesized phospholipid; and the like. As the test reagent for APTT measurement, a commercially available test reagent can be used. For example, Thrombocheck APTT series manufactured by Sysmex Corporation, Coagpia APTT-N of SEKISUI MEDICAL CO., LTD., Data phi APTT of Siemens Healthcare Diagnostics Products GmbH, and the like can be exemplified.

Calcium ions can be supplied by a 20 mM calcium chloride solution according to international standards.

Mixing of the test sample, the test reagent for APTT measurement and calcium ions is performed in a predetermined diluent. Examples of the diluent can include a solution that is isotonic with human plasma but does not have a pH adjusting function, like physiological saline, a buffer solution, and the like. Examples of the buffer solution can include Owren's veronal buffer solution and an imidazole buffer solution. As the diluent, Owren's veronal buffer is preferable.

In the above, detection of precipitation of fibrinogen is shown by the change in transmitted light intensity, but scattered light amount, absorbance and the like can be used instead of the transmitted light intensity.

APTT can also be measured using a blood coagulation measuring device. Examples of the blood coagulation measuring devices can include fully automated blood coagulation analyzers CN-6000, CN-3000, CS-2400, CS-2500, CS-5100, CS-1600, and CS-600 series; and semi-automated blood coagulation analyzers CA-101 and CA-104 manufactured by Sysmex Corporation. In this embodiment, since it is necessary to acquire a parameter related to differentiation of coagulation waveform, it is preferable to use a fully automated blood coagulation analyzer equipped with a software for differentiating coagulation waveform.

The activity value of factor VIII is acquired based on a calibration curve prepared from a plurality of standard plasma samples in which the activity value of factor VIII is known and the activity values are different from each other. According to the conventional method, the activity value of factor VIII using APTT is acquired based on a calibration curve of the activity value of factor VIII prepared from parameters related to coagulation activities acquired from the test sample and parameters corresponding to the parameters related to coagulation activities. The calibration curve is prepared from parameters related to coagulation activities acquired by APTT measurement of standard plasma samples obtained by diluting standard human plasma for blood coagulation test with physiological saline, factor VIII deficient plasma or the like in multiple stages. When standard human plasma for blood coagulation test is used to prepare a calibration curve, a sample obtained by diluting standard human plasma for blood coagulation test with physiological saline or factor VIII deficient plasma is used as a standard plasma sample with a known activity value of factor VIII, so that the activity value is at least one selected from, for example, theoretically 90%, 80%, 70%, 60%, 50%, 40%, 30%, 20%, and 10%, assuming that the factor VIII activity of standard human plasma for blood coagulation test is 100%. The standard plasma sample may include standard human plasma for blood coagulation test as a standard plasma sample with an activity value of factor VIII of 100%. The standard plasma sample may include factor VIII deficient plasma as a standard plasma sample with a factor VIII activity value of 0%. In this way, a plurality of standard plasma samples having factor VIII activity values different from each other can be prepared from the standard human plasma for blood coagulation test.

The standard human plasma for blood coagulation test may be pool plasma of plasma collected from a plurality of humans having no abnormality in blood coagulation function, and standard human plasma for blood coagulation test sold by Siemens K.K. or the like may be used. Factor VIII deficient plasma can be purchased, for example, from George King Bio-Medical, Inc. (USA) and the like.

For example, a standard plasma sample may be prepared by adding a blood coagulation factor VIII preparation such as CROSS EIGHT MC (Japan Blood Products Organization), KOVALTRY (trademark) (Bayer Yakuhin, Ltd.), ADVATE (Takeda Pharmaceutical Company Limited), ADYNOVATE (trademark) (Takeda Pharmaceutical Company Limited), NovoEight (trademark) (Novo Nordisk Pharma Ltd.) or ELOCTATE (trademark) (Sanofi K.K.) to factor VIII deficient plasma. Since the activity value of factor VIII is known for the blood coagulation factor VIII preparation, the blood coagulation factor VIII preparation is added to the factor VIII deficient plasma so as to obtain a desired activity value, and a plurality of standard plasma samples in which the activity values are different from each other can be prepared.

The parameter related to coagulation activity used to acquire the activity value of factor VIII is generally coagulation time. However, in this embodiment, the parameter related to differentiation of coagulation waveform is used to acquire the activity value of factor VIII. The parameter related to differentiation of coagulation waveform is obtained by performing differential processing on the coagulation waveform (hereinafter referred to as "original coagulation waveform") obtained by monitoring optical change of the reaction solution with time in APTT measurement. Differential processing is described in U.S. Patent Application No. 2018/0306820 and is incorporated herein by reference.

For example, a first differential coagulation waveform obtained by performing first differentiation on the original coagulation waveform is a parameter of coagulation rate. An apex of a peak of the first differential coagulation waveform indicates a maximum coagulation rate. In the present specification, the maximum coagulation rate may be expressed as "min1". A value of the first differential coagulation waveform of the coagulation rate may be represented by an absolute value, in which case the maximum coagulation rate can be represented by "|min1|".

A second differential coagulation waveform is obtained by performing secondary differential processing on the original coagulation waveform. The second differential coagulation waveform is parameters of coagulation acceleration and coagulation deceleration. In the second differential coagulation waveform, an apex of a peak obtained in a coaxial direction with the peak of the first differential coagulation waveform indicates maximum coagulation acceleration. In the present specification, the maximum coagulation acceleration may be expressed as "min2". Also, the maximum coagulation acceleration may be represented by an absolute value, in which case it can be represented by "|min2|". In the second differential coagulation waveform, a peak appearing in an axial direction opposite to an axial direction indicating acceleration indicates maximum coagulation deceleration. In the present specification, the maximum coagulation deceleration may be expressed as "max2". Also, the maximum coagulation deceleration may be represented by an absolute value, in which case it can be represented by "|max2|".

The first differentiation described above can also be performed on a corrected original coagulation waveform. In the present specification, the first differential coagulation waveform generated from the corrected coagulation waveform is referred to as a corrected first differential coagulation waveform. An apex of a peak of the corrected first differential coagulation waveform indicates a corrected maximum coagulation rate. In the present specification, the corrected maximum coagulation rate may be expressed as "Ad|min1|".

A corrected second differential coagulation waveform can be obtained by performing secondary differential processing on the corrected original coagulation waveform. In the corrected second differential coagulation waveform, an apex of a peak obtained in a coaxial direction with the peak of the corrected first differential coagulation waveform indicates corrected maximum coagulation acceleration. In the present specification, the maximum coagulation acceleration may be expressed as "Ad|min2|". In the corrected second differential coagulation waveform, a peak appearing in an axial direction opposite to an axial direction indicating acceleration indicates corrected maximum coagulation deceleration. In the present specification, the maximum coagulation deceleration may be expressed as "Ad|max2|".

The parameter related to differentiation of coagulation waveform may be any parameter that reflects shape of the first differential coagulation waveform, the corrected first differential coagulation waveform, the second differential coagulation waveform, or the corrected second differential coagulation waveform. For example, in this embodiment, a calibration curve is prepared using at least one parameters related to differentiation of coagulation waveform selected from the group consisting of a maximum coagulation rate, a maximum coagulation acceleration, a maximum coagulation deceleration, a corrected maximum coagulation rate, a corrected maximum coagulation acceleration, and a corrected maximum coagulation deceleration. Areas under curves of the first differential coagulation waveform, the corrected first differential coagulation waveform, the second differential coagulation waveform and the corrected second differential coagulation waveform, gravity center positions of regions under curves and the like are also used as parameters related to differentiation of coagulation waveform. Then, values of the parameters related to differentiation of coagulation waveform corresponding to the calibration curve acquired from the test sample are applied to the prepared calibration curve to acquire the activity value of factor VIII in the test sample.

The calibration curve, the parameters related to differentiation of coagulation waveform, and the activity value of factor VIII contained in the test sample can be obtained by the software installed in the fully automated blood coagulation analyzer.

### EXAMPLES

This embodiment will be described below in more detail, with reference to examples. However, the present disclosure is not construed as being limited to this embodiment.

### 1. Materials and methods

### (1) Preparation of test sample

Each test sample was prepared by adding a commercially available factor VIII preparation to Congenital Factor VIII deficient plasma (George King Bio-Medical, Inc. (USA)), so that final activity values would be 0.05 IU/dL, 0.30 IU/dL, and 1.00 IU/dL, based on an activity value listed in a package insert for each drug.

The factor VIII preparations used are as follows. CROSS EIGHT MC is a plasma fractionation preparation, and the other preparations are recombinant preparations.
CROSS EIGHT MC (Japan Blood Products Organization)
KOVALTRY (trademark) (Octocog beta; Bayer Yakuhin, Ltd.)
ADVATE (Rurioctocog alfa; Takeda Pharmaceutical Company Limited)
ADYNOVATE (Trademark) (Rurioctocog alfa pegol; Takeda Pharmaceutical Company Limited)
NovoEight (Trademark) (Turoctocog alfa; Novo Nordisk Pharma Ltd.)
ELOCTATE (Trademark) (Efraloctocog alfa; Sanofi K.K.)
AFSTYLA (trademark) (Lonoctocog alfa; CSL Behring K.K.)

### (2) Measurement reagent

Following reagents were used for the measurement.
Thrombocheck APTT-SLA (Sysmex Corporation)
20 mM Calcium chloride solution (Sysmex Corporation)
Coagulation Factor VIII deficient plasma (Siemens K.K.)
Owren's veronal buffer (Siemens K.K.)
Standard human plasma for blood coagulation test (Siemens K.K.)

### (3) Measuring device and measuring protocol

The measurement was performed by default protocol using a fully automated blood coagulation analyzer CS-2400 (Sysmex Corporation). Activated partial thromboplastin time (APTT) was measured by one-step coagulation method by performing the following steps using a computer program mounted on the analyzer to acquire an activity value of factor VIII of the test sample.
STEP 1: Dilute an aspirated test sample 20-fold with Owren's veronal buffer and dispense 40 µL into a reaction cuvette
STEP 2: Add 40 µL of coagulation factor VIII deficient plasma to the diluted test sample and incubate the mixture to prepare a first reaction solution
STEP 3: Add 40 µL of Thrombocheck APTT-SLA to the prepared first reaction solution and incubate the mixture to prepare a second reaction solution
STEP 4: Add 40 µL of a 20 mM calcium chloride solution to the second reaction solution to start a coagulation reaction, measure transmitted light at a wavelength of 660 nm for a predetermined time, and monitor the change in transmitted light intensity with time
STEP 5: Detect coagulation start point and coagulation end point from monitoring data and calculate coagulation time (CT)
STEP 6: Apply the calculated coagulation time (CT) to a calibration curve for factor VIII to calculate coagulation factor VIII activity of a specimen

The calibration curve for factor VIII was prepared using standard plasma samples of standard human plasma for blood coagulation test diluted stepwise with coagulation factor VIII deficient plasma. At this time, the activity value of factor VIII of undiluted standard human plasma for blood coagulation test was set to 100%. The standard plasma sample was also measured in the same manner as the test sample, and a calibration curve was prepared with the coagulation time as the factor VIII activity according to a dilution ratio.

### (4) Acquisition of parameters related to differentiation of APTT coagulation waveform

As parameters related to differentiation of APTT coagulation waveform, min1, min2, Ad|min1|, and max2 were calculated. The parameters related to differentiation of APTT coagulation waveform were calculated for the test sample and the standard plasma sample.

The monitoring data obtained in (3) STEP 4 above was used as APTT coagulation waveform data. The APTT coagulation waveform was obtained by plotting the monitoring data with X axis as measurement time and Y axis as transmitted light intensity.

The APTT coagulation waveform was differentiated according to a method described in U.S. Patent Application No. 2018/0306820 to generate a first differential coagulation waveform and a second differential coagulation waveform of the APTT coagulation waveform. Peak value min1 of the velocity waveform was further obtained from the first differential coagulation waveform. min1 indicates a maximum coagulation rate. Peak value min2 of coagulation acceleration and absolute value max2 of peak value of coagulation deceleration were obtained from the second differential coagulation waveform. min2 indicates a maximum coagulation acceleration. max2 indicates a maximum coagulation deceleration. Absolute value |min1| of peak value of the velocity waveform was further obtained from a corrected first differential coagulation waveform generated based on normalized APTT coagulation waveform data, and taken as corrected absolute value Ad|min1|. A corrected first differential coagulation waveform and a corrected second differential coagulation waveform were generated from the normalized APTT coagulation waveform.

Based on the parameters related to differentiation of APTT coagulation waveform obtained from the standard plasma sample, a calibration curve of the activity value of factor VIII based on each parameter was prepared. Based on this calibration curve, activity values of factor VIII of each test sample were calculated for each parameter.

### 2. Results

### (1) Comparison of factor VIII activity of each preparation

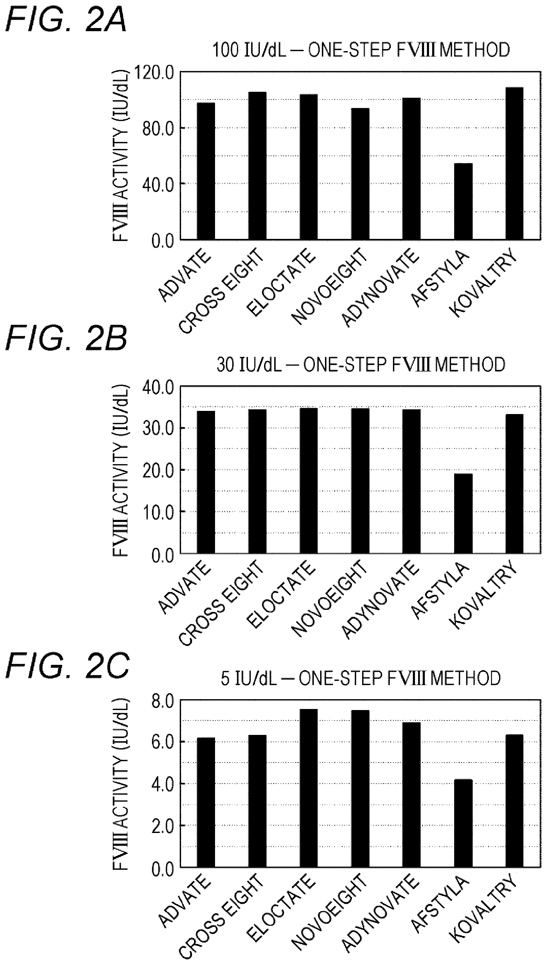

Figs. 2A to 2C show the activity values (IU/dL) of factor VIII of each preparation calculated based on the coagulation time (CT) obtained by one-step APTT method. Measurement was performed on each test sample n = 3. Fig. 2A shows the activity values when each preparation was added to Congenital Factor VIII deficient plasma so as to be 1.00 IU/dL. Fig. 2B shows the activity values when each preparation was added to Congenital Factor VIII deficient plasma so as to be 0.30 IU/dL. Fig. 2C shows the activity values when each preparation was added to Congenital Factor VIII deficient plasma so as to be 0.05 IU/dL. Only AFSTYLA showed low value in all activities.

### (2) Data comparison between parameters related to differentiation of APTT coagulation waveform

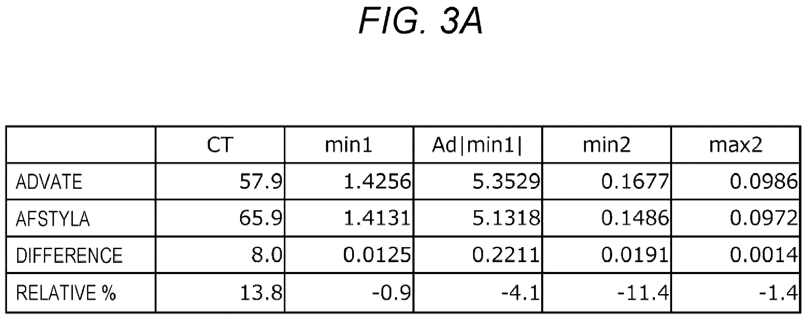

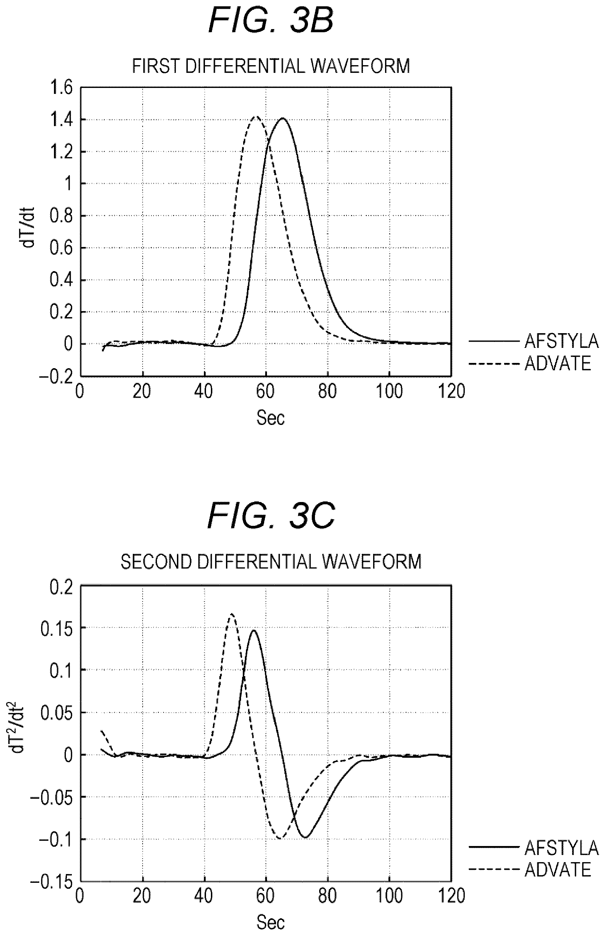

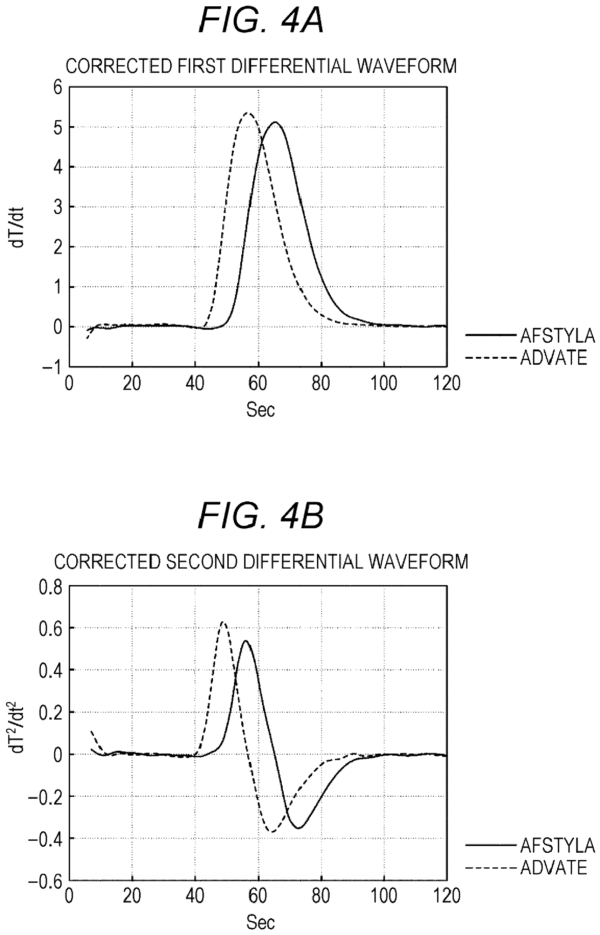

Fig. 3A shows parameter values of ADVATE and AFSTYLA, which had largest difference in coagulation time (CT) in the result of (1) above. Unit of CT is seconds, and other data indicate calculated values. Fig. 3B shows first differential waveforms of APTT coagulation waveforms of both. Fig. 3C shows second differential waveforms of APTT coagulation waveforms of both. Fig. 4A shows first differential waveforms of corrected APTT coagulation waveforms of both. Fig. 4B shows secondary differential waveforms of corrected APTT coagulation waveforms of both. In the first differential waveform, Y axis is represented by coagulation rate (dT/dt). Here, T is time when maximum transmitted light intensity was measured, and t is time when minimum transmitted light intensity was measured. In the second differential waveform, Y axis is represented by coagulation acceleration (dT²/dt²).

As shown in Fig. 3A, CT was longer in AFSTYLA, which indicated that activity of factor VIII was lower. However, when comparing min1, Ad|min1|, and max2, it has been shown that difference in factor VIII activity values between ADVATE and AFSTYLA is smaller than difference in factor VIII activity values between ADVATE and AFSTYLA when comparing CT.

### (3) Calibration curve for each parameter related to differentiation of APTT coagulation waveform

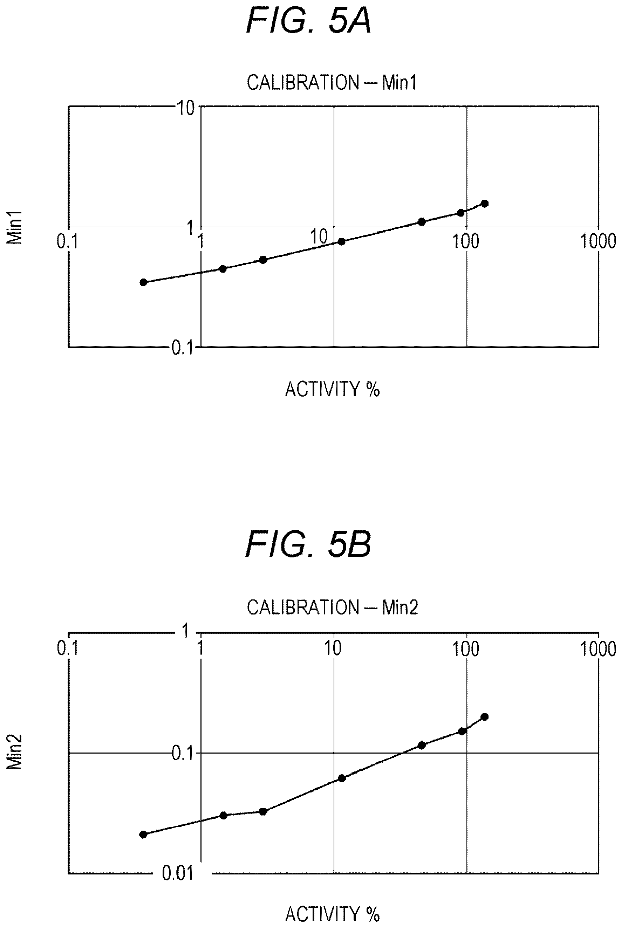

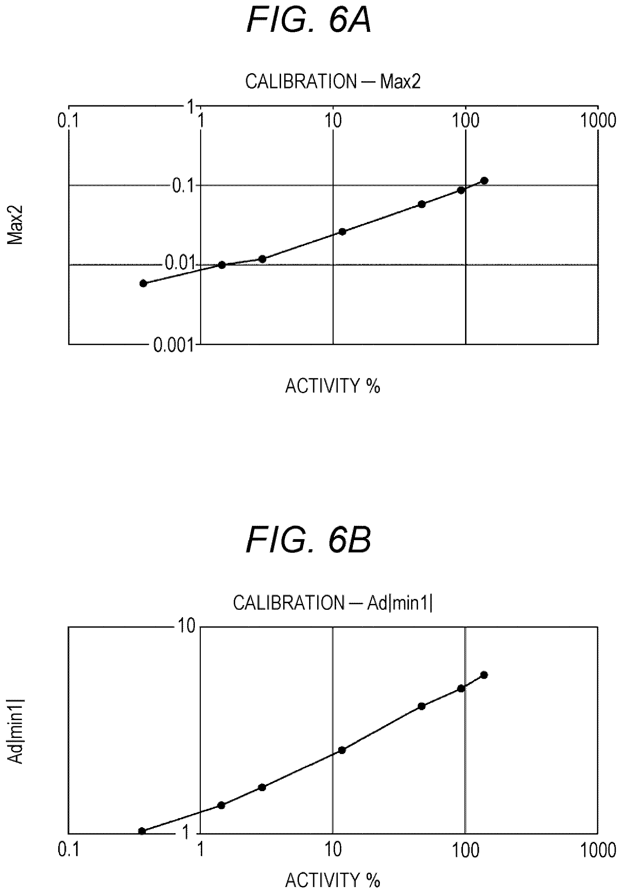

Next, calibration curves prepared for each parameter related to differentiation of APTT coagulation waveform are shown in Figs. 5A and 5B and Figs. 6A and 6B. Fig. 5A is a calibration curve of min1, and Fig. 5B is a calibration curve of min2. Fig. 6A is a calibration curve of Ad|min1|, and Fig. 6B is a calibration curve of Max2. Each axis of the calibration curves is represented by a logarithmic scale. Y axis shows values of each parameter, and X axis shows activity values of factor VIII.

All the calibration curves of parameters had good linearity, indicating that they can evaluate the activity value of factor VIII.

### (4) Calculation of activity values of AFSTYLA using parameters related to differentiation of APTT coagulation waveform

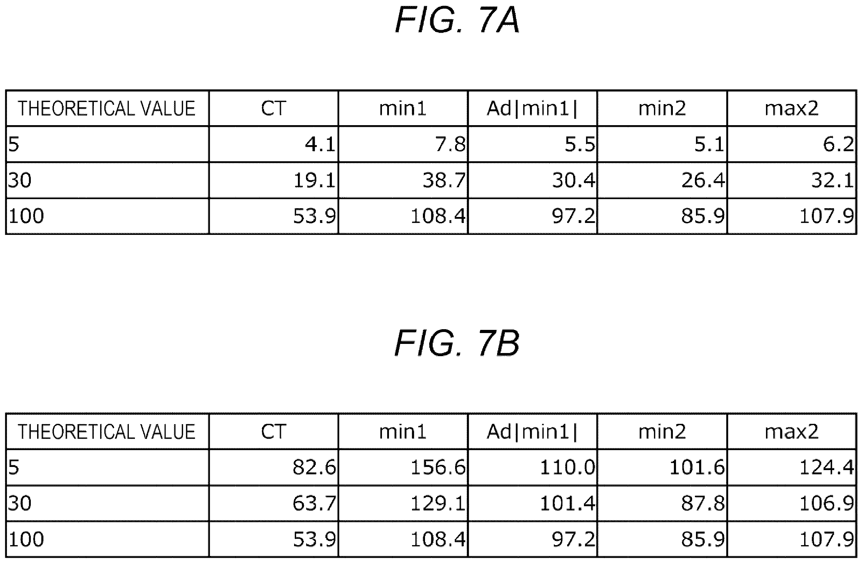

APTT coagulation data was acquired by adding AFSTYLA to Congenital Factor VIII deficient plasma, so that final activity values would be 5 IU/dL, 30 IU/dL, and 100 IU/dL, based on an activity value listed in a package insert. Parameter values related to differentiation of each APTT coagulation waveform were acquired based on this data, and applied to the calibration curve to calculate activity values of factor VIII. The results are shown in Figs. 7A and 7B. Fig. 7A shows the activity values of factor VIII, and Fig. 7B shows addition recovery rates (%). The activity values of factor VIII of AFSTYLA calculated using the parameters related to differentiation of APTT coagulation waveform showed higher values than the activity values calculated from CT, and showed values close to theoretical values. The recovery rates were also good.

### (5) Factor VIII activities calculated using parameters related to differentiation of APTT coagulation waveform

For the test samples showing activities in Figs. 2A to 2C, activity values of factor VIII were calculated using the parameters related to differentiation of APTT coagulation waveform.

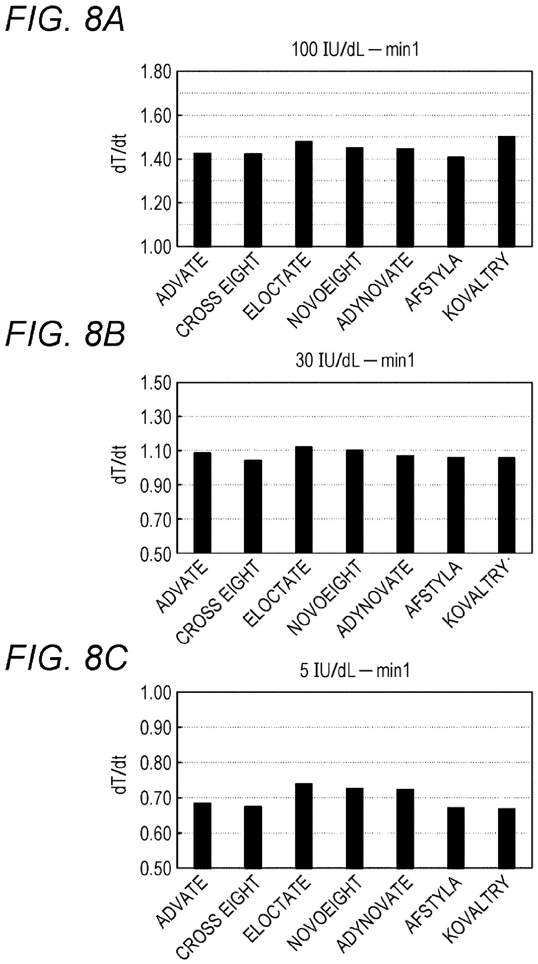

Fig. 8A to 8C show activity values of factor VIII of each preparation calculated based on maximum coagulation rate min1. Fig. 8A shows the activity values when each preparation was added to Congenital Factor VIII deficient plasma so as to be 1.00 IU/dL. Fig. 8B shows the activity values when each preparation was added to Congenital Factor VIII deficient plasma so as to be 0.30 IU/dL. Fig. 8C shows the activity values when each preparation was added to Congenital Factor VIII deficient plasma so as to be 0.05 IU/dL.

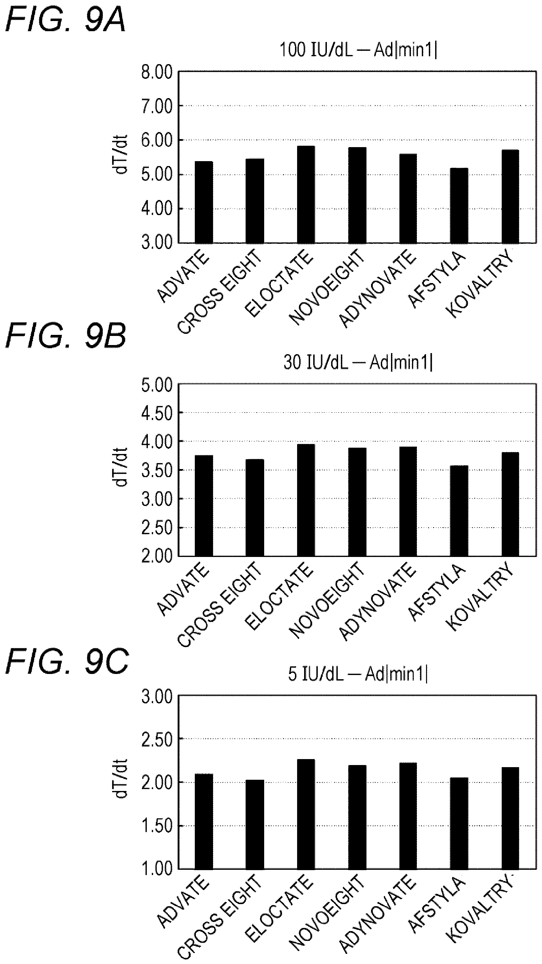

Figs. 9A to 9C show activity values of factor VIII of each preparation calculated based on corrected maximum coagulation rate Ad|min1|. Fig. 9A shows the activity values when each preparation was added to Congenital Factor VIII deficient plasma so as to be 1.00 IU/dL. Fig. 9B shows the activity values when each preparation was added to Congenital Factor VIII deficient plasma so as to be 0.30 IU/dL. Fig. 9C shows the activity values when each preparation was added to Congenital Factor VIII deficient plasma so as to be 0.05 IU/dL.

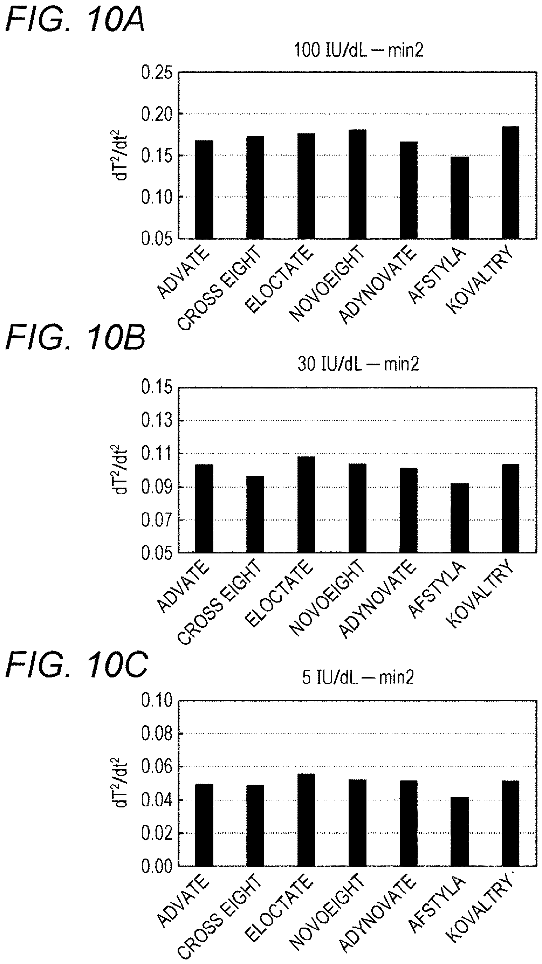

Figs. 10A to 10C show activity values of factor VIII of each preparation calculated based on maximum coagulation acceleration min2. Fig. 10A shows the activity values when each preparation was added to Congenital Factor VIII deficient plasma so as to be 1.00 IU/dL. Fig. 10B shows the activity values when each preparation was added to Congenital Factor VIII deficient plasma so as to be 0.30 IU/dL. Fig. 10C shows the activity values when each preparation was added to Congenital Factor VIII deficient plasma so as to be 0.05 IU/dL.

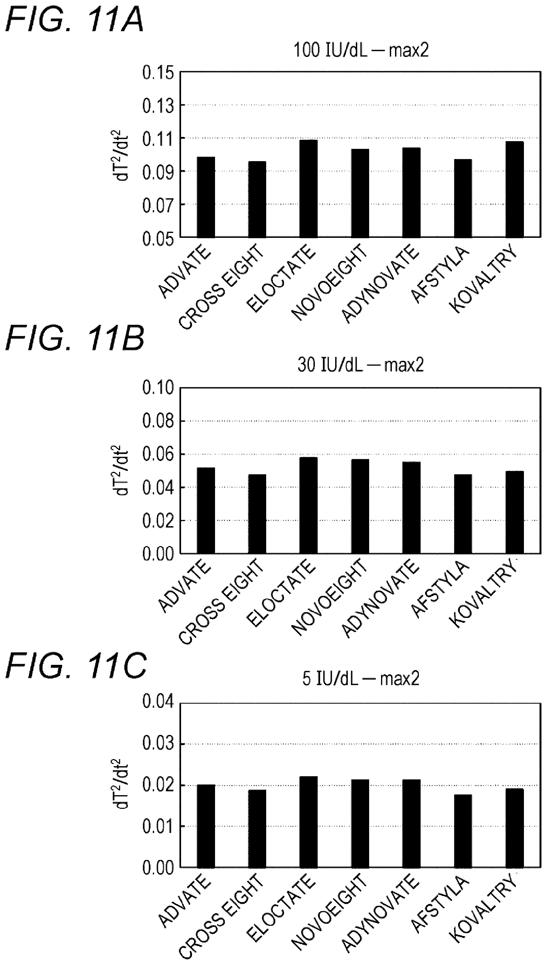

Figs. 11A to 11C show activity values of factor VIII of each preparation calculated based on maximum coagulation deceleration max2. Fig. 11A shows the activity values when each preparation was added to Congenital Factor VIII deficient plasma so as to be 1.00 IU/dL. Fig. 11B shows the activity values when each preparation was added to Congenital Factor VIII deficient plasma so as to be 0.30 IU/dL. Fig. 11C shows the activity values when each preparation was added to Congenital Factor VIII deficient plasma so as to be 0.05 IU/dL.

FIGS.2A to 2C, 9A to 11C show the activity values of factor VIII of AFSTYLA calculated based on a parameter related to differentiation of coagulation waveform are higher than the activity values of factor VIII calculated based on the CT, and difference in activity values from other factor VIII preparations was eliminated.

From this, it was shown that it is useful to use a parameter related to differentiation of coagulation waveform instead of using a CTin determining an administration effect of AFSTYLA (Lonoctocog alfa).

In addition, regarding the obtained activity value, it is not necessary to specify a specimen of a patient to whom AFSTYLA (Lonoctocog alfa) has been administered, which can facilitate the measurement and also suppress human error.

## Claims

1. A method for measuring a blood coagulation activity, comprising:
acquiring a parameter related to differentiation of a coagulation waveform in a blood specimen collected from a patient administered with
(1) a polypeptide containing a sequence represented by SEQ ID NO: 1, or
(2) a polypeptide containing a polypeptide having 95% or more identity with the sequence represented by SEQ ID NO: 1 and having an activity as a blood coagulation factor VIII; and
acquiring an activity value of the blood coagulation factor VIII in the blood specimen based on the acquired parameter.

2. The method for measuring the blood coagulation activity according to claim 1, wherein the parameter is acquired based on the coagulation waveform measured by one-step coagulation method.

3. The method for measuring the blood coagulation activity according to claim 2, wherein the measurement by one-step coagulation method is measurement of activated partial thromboplastin time.

4. The method for measuring the blood coagulation activity according to any one of claims 1 to 3, wherein the parameter is a parameter acquired from at least one selected from a waveform of a coagulation rate obtained by firstly differentiating the coagulation waveform and a waveform of a coagulation acceleration obtained by secondary differentiating the coagulation waveform.

5. The method for measuring the blood coagulation activity according to claim 4, wherein the parameter is a value indicating at least one selected from a maximum coagulation rate, a maximum coagulation acceleration, a maximum coagulation deceleration, a corrected maximum coagulation rate, a corrected maximum coagulation acceleration, and a corrected maximum coagulation deceleration.

6. The method for measuring the blood coagulation activity according to any one of claims 1 to 5, wherein the activity value of the blood coagulation factor VIII in the blood specimen collected from the patient is acquired based on a calibration curve prepared from a plurality of standard plasma samples in which an activity value of the coagulation factor VIII is known and the activity values of the blood coagulation factor VIII are different from each other.


SEQUENCE LISTING

<110>  KEIO UNIVERSITY
       TOKYO SAISEIKAI CENTRAL HOSPITAL
       SYSMEX CORPORATION

<120>  A method for measuring blood coagulation activity

<130>  19-048JP

<160>  1

<170>  PatentIn version 3.5

<210>  1
<211>  1444
<212>  PRT
<213>  Artificial Sequence

<220>
<223>  Recombinant Factor VIII

<400>  1

Ala Thr Arg Arg Tyr Tyr Leu Gly Ala Val Glu Leu Ser Trp Asp Tyr
1               5                   10                  15

Met Gln Ser Asp Leu Gly Glu Leu Pro Val Asp Ala Arg Phe Pro Pro
            20                  25                  30

Arg Val Pro Lys Ser Phe Pro Phe Asn Thr Ser Val Val Tyr Lys Lys
        35                  40                  45

Thr Leu Phe Val Glu Phe Thr Val His Leu Phe Asn Ile Ala Lys Pro
    50                  55                  60

Arg Pro Pro Trp Met Gly Leu Leu Gly Pro Thr Ile Gln Ala Glu Val
65                  70                  75                  80

Tyr Asp Thr Val Val Ile Thr Leu Lys Asn Met Ala Ser His Pro Val
                85                  90                  95

Ser Leu His Ala Val Gly Val Ser Tyr Trp Lys Ala Ser Glu Gly Ala
            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn
    130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg
225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr
            420                 425                 430

Asp Glu Thr Phe Lys Thr Arg Glu Ala Ile Gln His Glu Ser Gly Ile
        435                 440                 445

Leu Gly Pro Leu Leu Tyr Gly Glu Val Gly Asp Thr Leu Leu Ile Ile
    450                 455                 460

Phe Lys Asn Gln Ala Ser Arg Pro Tyr Asn Ile Tyr Pro His Gly Ile
465                 470                 475                 480

Thr Asp Val Arg Pro Leu Tyr Ser Arg Arg Leu Pro Lys Gly Val Lys
                485                 490                 495

His Leu Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe
                565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro

            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Pro Ser Thr Arg
            740                 745                 750

Gln Lys Gln Phe Asn Ala Thr Thr Ile Pro Glu Asn Thr Thr Leu Gln
        755                 760                 765

Ser Asp Gln Glu Glu Ile Asp Tyr Asp Asp Thr Ile Ser Val Glu Met
    770                 775                 780

Lys Lys Glu Asp Phe Asp Ile Tyr Asp Glu Asp Glu Asn Gln Ser Pro
785                 790                 795                 800

Arg Ser Phe Gln Lys Lys Thr Arg His Tyr Phe Ile Ala Ala Val Glu
                805                 810                 815

Arg Leu Trp Asp Tyr Gly Met Ser Ser Ser Pro His Val Leu Arg Asn
            820                 825                 830

Arg Ala Gln Ser Gly Ser Val Pro Gln Phe Lys Lys Val Val Phe Gln
        835                 840                 845

Glu Phe Thr Asp Gly Ser Phe Thr Gln Pro Leu Tyr Arg Gly Glu Leu
    850                 855                 860

Asn Glu His Leu Gly Leu Leu Gly Pro Tyr Ile Arg Ala Glu Val Glu
865                 870                 875                 880

Asp Asn Ile Met Val Thr Phe Arg Asn Gln Ala Ser Arg Pro Tyr Ser
                885                 890                 895

Phe Tyr Ser Ser Leu Ile Ser Tyr Glu Glu Asp Gln Arg Gln Gly Ala
            900                 905                 910

Glu Pro Arg Lys Asn Phe Val Lys Pro Asn Glu Thr Lys Thr Tyr Phe
        915                 920                 925

Trp Lys Val Gln His His Met Ala Pro Thr Lys Asp Glu Phe Asp Cys
    930                 935                 940

Lys Ala Trp Ala Tyr Phe Ser Asp Val Asp Leu Glu Lys Asp Val His
945                 950                 955                 960

Ser Gly Leu Ile Gly Pro Leu Leu Val Cys His Thr Asn Thr Leu Asn
                965                 970                 975

Pro Ala His Gly Arg Gln Val Thr Val Gln Glu Phe Ala Leu Phe Phe
            980                 985                 990

Thr Ile Phe Asp Glu Thr Lys Ser Trp Tyr Phe Thr Glu Asn Met Glu
        995                 1000                1005

Arg Asn Cys Arg Ala Pro Cys Asn Ile Gln Met Glu Asp Pro Thr
    1010                1015                1020

Phe Lys Glu Asn Tyr Arg Phe His Ala Ile Asn Gly Tyr Ile Met
    1025                1030                1035

Asp Thr Leu Pro Gly Leu Val Met Ala Gln Asp Gln Arg Ile Arg
    1040                1045                1050

Trp Tyr Leu Leu Ser Met Gly Ser Asn Glu Asn Ile His Ser Ile
    1055                1060                1065

His Phe Ser Gly His Val Phe Thr Val Arg Lys Lys Glu Glu Tyr
    1070                1075                1080

Lys Met Ala Leu Tyr Asn Leu Tyr Pro Gly Val Phe Glu Thr Val
    1085                1090                1095

Glu Met Leu Pro Ser Lys Ala Gly Ile Trp Arg Val Glu Cys Leu
    1100                1105                1110

Ile Gly Glu His Leu His Ala Gly Met Ser Thr Leu Phe Leu Val
    1115                1120                1125

Tyr Ser Asn Lys Cys Gln Thr Pro Leu Gly Met Ala Ser Gly His
    1130                1135                1140

Ile Arg Asp Phe Gln Ile Thr Ala Ser Gly Gln Tyr Gly Gln Trp
    1145                1150                1155

Ala Pro Lys Leu Ala Arg Leu His Tyr Ser Gly Ser Ile Asn Ala
    1160                1165                1170

Trp Ser Thr Lys Glu Pro Phe Ser Trp Ile Lys Val Asp Leu Leu
    1175                1180                1185

Ala Pro Met Ile Ile His Gly Ile Lys Thr Gln Gly Ala Arg Gln
    1190                1195                1200

Lys Phe Ser Ser Leu Tyr Ile Ser Gln Phe Ile Ile Met Tyr Ser
    1205                1210                1215

Leu Asp Gly Lys Lys Trp Gln Thr Tyr Arg Gly Asn Ser Thr Gly
    1220                1225                1230

Thr Leu Met Val Phe Phe Gly Asn Val Asp Ser Ser Gly Ile Lys
    1235                1240                1245

His Asn Ile Phe Asn Pro Pro Ile Ile Ala Arg Tyr Ile Arg Leu
    1250                1255                1260

His Pro Thr His Tyr Ser Ile Arg Ser Thr Leu Arg Met Glu Leu
    1265                1270                1275

Met Gly Cys Asp Leu Asn Ser Cys Ser Met Pro Leu Gly Met Glu
    1280                1285                1290

Ser Lys Ala Ile Ser Asp Ala Gln Ile Thr Ala Ser Ser Tyr Phe
    1295                1300                1305

Thr Asn Met Phe Ala Thr Trp Ser Pro Ser Lys Ala Arg Leu His
    1310                1315                1320

Leu Gln Gly Arg Ser Asn Ala Trp Arg Pro Gln Val Asn Asn Pro
    1325                1330                1335

Lys Glu Trp Leu Gln Val Asp Phe Gln Lys Thr Met Lys Val Thr
    1340                1345                1350

Gly Val Thr Thr Gln Gly Val Lys Ser Leu Leu Thr Ser Met Tyr
    1355                1360                1365

Val Lys Glu Phe Leu Ile Ser Ser Ser Gln Asp Gly His Gln Trp
    1370                1375                1380

Thr Leu Phe Phe Gln Asn Gly Lys Val Lys Val Phe Gln Gly Asn
    1385                1390                1395

Gln Asp Ser Phe Thr Pro Val Val Asn Ser Leu Asp Pro Pro Leu
    1400                1405                1410

Leu Thr Arg Tyr Leu Arg Ile His Pro Gln Ser Trp Val His Gln
    1415                1420                1425

Ile Ala Leu Arg Met Glu Val Leu Gly Cys Glu Ala Gln Asp Leu
    1430                1435                1440

Tyr